(19)

Europäisches Patentamt
European Patent Office
Office européen des brevets

(11) **EP 4 502 577 A1**

(12) **EUROPEAN PATENT APPLICATION**
published in accordance with Art. 153(4) EPC

(43) Date of publication:
**05.02.2025 Bulletin 2025/06**

(21) Application number: **23784510.2**

(22) Date of filing: **31.01.2023**

(51) International Patent Classification (IPC):
$G01N\ 21/27$ (2006.01)   $C12M\ 1/34$ (2006.01)
$C12Q\ 1/06$ (2006.01)   $G01J\ 3/46$ (2006.01)
$G01N\ 33/53$ (2006.01)   $G01N\ 33/574$ (2006.01)

(52) Cooperative Patent Classification (CPC):
C12M 1/34; C12Q 1/06; G01J 3/46; G01N 21/27;
G01N 33/53; G01N 33/574

(86) International application number:
**PCT/JP2023/003087**

(87) International publication number:
**WO 2023/195215 (12.10.2023 Gazette 2023/41)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.04.2022 JP 2022064581**

(71) Applicant: **Sumitomo Chemical Company, Limited
Tokyo 103-6020 (JP)**

(72) Inventors:
• **KAWABATA Ryota
Niihama-shi, Ehime 792-0015 (JP)**
• **KANO Hanaho
Osaka-shi, Osaka 554-8558 (JP)**
• **OZAKI Maya
Niihama-shi, Ehime 792-8521 (JP)**

(74) Representative: **Winter, Brandl - Partnerschaft
mbB
Alois-Steinecker-Straße 22
85354 Freising (DE)**

(54) **TRANSPARENCY EVALUATION METHOD**

(57)   A transparency assessment method according to one embodiment includes: a step of acquiring N frequency distributions for a target color in N predetermined regions in a plate image, the N frequency distributions having at least two color feature quantities as variates; and a step of assessing transparency based on a first reference frequency distribution for a first reference target color which is a target color of a first transparent state, and based on the N frequency distributions. The plate image is an image corresponding to an assessment plate in which an assessment target including a tester is held in a plurality of wells. The plurality of wells have at least one test substance well that holds the assessment target including the tester. At least one of the N predetermined regions includes an image corresponding to the test substance well. In the step of assessing the transparency, the transparency is assessed based on a centroid vector directed from a centroid position of a first reference target color region in the first reference frequency distribution toward a centroid position of a target color region in the N frequency distributions.

*Fig.13*

# Description

## Technical Field

The present invention relates to a transparency assessment method.

## Background Art

As an example of biological safety assessment, there is a method in which a test substance is added to a tester, and for example, a change in transparency is observed to assess whether or not the test substance is toxic to the tester. In recent years, in order to reduce the amounts of the tester and the test substance, a method using such a plate having a plurality of wells as disclosed, for example, in Patent Literature 1, Patent Literature 2, Non-Patent Literature 1, Non-Patent Literature 2, and Non-Patent Literature 3 may be adopted.

## Citation List

## Patent Literature

[Patent Literature 1] Japanese Unexamined Patent Application Publication No. 2017-67605
[Patent Literature 2] Japanese Unexamined Patent Application Publication No. 2017-85966

## Non-Patent Literature

[Non-Patent Literature 1] Fluckiger-Isler S., Kamber M., "Direct comparison of the Ames microplate format (MPF) test in liquid medium with the standard Ames pre-incubation assay on agar plates by use of equivocal to weakly positive test compounds.", Mutation Research/Genetic Toxicology and Environmental Mutagenesis, 747, p36-45,2012.
[Non-Patent Literature 2] Sui H., Kawakami K., Sakurai N., Hara T., Nohmi T., "Improvement and evaluation of high throughput fluctuation Ames test using 384-well plate with Salmonella typhimurium TA100 and TA98.", Genes and Environment 31(2), p47-55, 2009.
[Non-Patent Literature 3] Kamber M., Fluckiger-Isler S., Engelhardt G., Jaeckh R., Zeiger E., "Comparison of the Ames II and traditional Ames test responses with respect to mutagenicity, strain specificities, need for metabolism and correlation with rodent carcinogenicity", Mutagenesis 24(4), p359-366, 2009.

# Summary of Invention

## Technical Problem

As disclosed in Patent Literature 1, Patent Literature 2, and the like, in a method of assessing a tester or a test substance based on a color change using a plate having a plurality of wells, there is a demand for an assessment method for color change, which is more objective and can improve accuracy. The transparency change in the Ames test is one kind of such color change and is used to assess the effect of the tester on the test substance. In addition, there is a demand for a transparency assessment method, which is more objective and can improve accuracy.

Therefore, an object of the present invention is to provide a transparency assessment method that enables objective assessment and can improve accuracy.

## Solution to Problem

A transparency assessment method according to the present invention includes the following aspects [1] to [8].

[1] A transparency assessment method comprising:

a step of acquiring N frequency distributions (N is an integer of 1 or more) for a target color in N predetermined regions in a plate image, the N frequency distributions having at least two color feature quantities as variates; and
a step of assessing transparency of the N predetermined regions based on a first reference frequency distribution for a first reference target color and the N frequency distributions, the first reference frequency distribution having the at least two color feature quantities as variates,
wherein the plate image is an image corresponding to an assessment plate in which an assessment target is held in a plurality of wells provided in the plate,
the assessment target includes a tester,
the plurality of wells have at least one test substance well that holds the assessment target further including a test substance,
at least one of the N predetermined regions includes at least one image corresponding to the at least one test substance well,
the first reference target color is the target color that is in a first transparent state, and
in the step of assessing the transparency, the transparency is assessed based on a centroid vector directed from a first centroid position of a first reference target color region in the first reference frequency distribution toward a second centroid position of a target color region in each of the N frequency distributions.

In the above method, the transparency of N predetermined regions can be assessed based on N frequency distributions obtained from the plate image. Therefore, objectivity can be ensured, for example, as compared with a case of visually assessing transparency. In the step of assessing the transparency, the transparency is assessed based on the centroid vector directed from the first centroid position of the first reference target color region in the first reference frequency distribution corresponding to the first reference target color that is a color corresponding to the first transparent state of the target color toward a second centroid position of a target color region in each of the N frequency distributions. Therefore, the accuracy of transparency assessment can be improved.

[2] The transparency assessment method according to [1], further comprising:

    a step of acquiring the first reference frequency distribution,
    wherein the plurality of wells further include at least one first reference target color well for the first reference target color,
    one of the N predetermined regions includes an image corresponding to the at least one first reference target color well, and
    in the step of acquiring the first reference frequency distribution, a frequency distribution having the at least two color feature quantities as variates in a predetermined region including the image corresponding to the at least one first reference target color well among the N predetermined regions is acquired as the first reference frequency distribution.

In this case, the first reference frequency distribution serving as a reference for transparency assessment can be acquired based on the frequency distribution obtained from the plate image to be assessed. Therefore, the transparency can be assessed according to the plate image to be assessed, and thus it is easy to improve the accuracy of the transparency assessment.

[3] The transparency assessment method according to [1] or [2], wherein in the step of assessing the transparency, the transparency is assessed based on an angle of the centroid vector with respect to a transparency change reference vector and a length of the centroid vector,

    the transparency change reference vector is a vector directed from the first centroid position toward a third centroid position of a second reference target color region in a second reference frequency distribution for a second reference target color, the second reference frequency distribution having at least two color feature quantities as variates,
the second reference target color is the target color that is in a second transparent state different from the first transparent state, and
the length of the centroid vector is a length standardized by an image size in a case where a frequency distribution including the second centroid position defining the centroid vector is displayed as an image.

In this case, the transparency can be assessed using, as a reference, the transparency change from the first reference target color to the second reference target color.

[4] The transparency assessment method according to [3], further comprising:

    a step of acquiring the second reference frequency distribution,
    wherein in the step of acquiring the second reference frequency distribution, a frequency distribution having the at least two color feature quantities as variates for the second reference target color in one predetermined region among the N predetermined regions is acquired as the second reference frequency distribution.

In this case, the second reference frequency distribution that is used for defining a transparency change vector can be acquired based on the frequency distribution obtained from the plate image to be assessed. Therefore, the transparency can be assessed according to the plate image to be assessed, and thus it is easy to improve the accuracy of the transparency assessment.

[5] The transparency assessment method according to [1], wherein in the step of acquiring the N frequency distributions, in each of the N predetermined regions, frequency distributions for a first reference target color and a second reference target color are acquired, and a frequency distribution of the target color is acquired based on the frequency distribution for the first reference target color and the frequency distribution for the second reference target color,

    the second reference target color is the target color that is in a second transparent state different from the first transparent state,
    in the step of assessing the transparency, the transparency is assessed based on an angle of the centroid vector with respect to a transparency change reference vector and a length of the centroid vector,
    the transparency change reference vector is a vector directed from the first centroid position toward a third centroid position of a second reference target color region in a second reference frequency distribution, the second reference frequency distribution having at least two color feature quantities as variates,

the first reference frequency distribution is a frequency distribution for the first reference target color acquired in one predetermined region among the N predetermined regions,

the second reference frequency distribution is a frequency distribution for the second reference target color acquired in one predetermined region among the N predetermined regions, and

the length of the centroid vector is a length standardized by an image size in a case where a frequency distribution including the second centroid position defining the centroid vector is displayed as an image.

In this case, the transparency can be assessed using, as a reference, the transparency change from the first reference target color to the second reference target color. In the above method, the first reference frequency distribution and the second reference frequency distribution, which serve as a reference for transparency assessment, can be acquired based on the frequency distribution obtained from the plate image to be assessed. Therefore, the transparency can be assessed according to the plate image to be assessed, and thus it is easy to improve the accuracy of the transparency assessment.

[6] The transparency assessment method according to [5], wherein the step of acquiring the N frequency distributions includes:

a step of acquiring an initial frequency distribution having the at least two color feature quantities as variates with respect to a plurality of regions in each of the N predetermined regions;

a step of determining colors of the plurality of regions based on a degree of similarity between the initial frequency distribution with respect to the plurality of regions and a first frequency distribution model for the first reference target color and a degree of similarity between the initial frequency distribution with respect to the plurality of regions and a second frequency distribution model for the second reference target color; and

a step of acquiring frequency distributions for the first reference target color and the second reference target color by using an initial frequency distribution of a region determined to correspond to the first reference target color and the second reference target color, respectively, among the plurality of regions.

In this case, in each of the N predetermined regions, frequency distributions for the first reference target color and the second reference target color according to the state of each predetermined region can be acquired.

[7] The transparency assessment method according to [1], further comprising:

a step of shifting image data corresponding to a frequency distribution having the at least two color feature quantities as variates in a direction of one of the at least two color feature quantities, wherein in the step of acquiring the N frequency distributions, in each of the N predetermined regions, frequency distributions for a first reference target color, a second reference target color, and a comparative control color are acquired, and concurrently, a frequency distribution of the target color is acquired based on the frequency distribution for the first reference target color and the frequency distribution for the second reference target color,

the second reference target color is the target color that is in a second transparent state different from the first transparent state,

the comparative control color is a color different from the target color,

in the step of assessing the transparency, the transparency is assessed based on an angle of the centroid vector with respect to a transparency change reference vector, an angle of a centroid vector with respect to a color change reference vector, and a length of the centroid vector,

the transparency change reference vector is a vector directed from the first centroid position toward a third centroid position of a second reference target color region in a second reference frequency distribution,

the color change reference vector is a vector directed from the first centroid position toward a fourth centroid position of a comparative control color region in a comparative control frequency distribution,

the first reference frequency distribution is a frequency distribution for the first reference target color acquired in one predetermined region among the N predetermined regions,

the second reference frequency distribution is a frequency distribution for the second reference target color acquired in one predetermined region among the N predetermined regions,

the comparative control frequency distribution is a frequency distribution for the comparative control color acquired in one predetermined region among the N predetermined regions,

in the step of carrying out the shifting, image data corresponding to the N frequency distributions, the first reference frequency distribution, the second reference frequency distribution, and the comparative control frequency distribution are shifted in a direction of one of the at least two color feature quantities with respect to a display region in a case where the N frequency distributions, the first reference frequency distribution, the second reference frequency distribution, and the comparative control frequency distribution are displayed as images, and

each of the first centroid position, the second centroid position, the third centroid position, and the fourth centroid position is a centroid position based on image data corresponding to each of the N frequency distributions, the first reference frequency distribution, the second reference frequency distribution, and the comparative control frequency distribution, which are the shifted image data.

In the above method, in a case where there is a comparative control color different from the target color in the assessment plate to be subjected to transparency assessment, the transparency can be assessed based on the relationship between the transparency change reference vector and the color change reference vector.

[8] The transparency assessment method according to any one of [1] to [7], wherein the at least two color feature quantities include hue and saturation.

**Advantageous Effects of Invention**

According to the present invention, it is possible to provide a transparency assessment method that enables objective assessment and can improve accuracy.

**Brief Description of Drawings**

FIG. 1 is a plan view of an assessment plate that is used in an assessment method for an assessment target according to one embodiment.
FIG. 2 is a schematic view of a cross section of a well.
FIG. 3 is a schematic view of an assessment system for an assessment target according to one embodiment.
FIG. 4 is a drawing showing an example of an image of an assessment plate (plate image).
FIG. 5 is a drawing showing a flowchart of an example of a transparency assessment method.
FIG. 6 is a drawing showing an example of an HSV image.
FIG. 7 is a drawing showing a histogram of an example of a frequency distribution created for wells.
FIG. 8 is a drawing showing an example of an image corresponding to a frequency distribution for a first reference target color.
FIG. 9 is a drawing showing an example of an image corresponding to a frequency distribution for a second reference target color.

FIG. 10 is a drawing showing an example of an image corresponding to a frequency distribution for yellow (comparative control color).
FIG. 11 is a drawing for an explanatory description of a step of shifting image data in a case where a frequency distribution is displayed as an image.
FIG. 12 is a drawing showing the image shown in FIG. 8 that has been shifted by the step of shifting the image data in a case where the frequency distribution is displayed as an image.
FIG. 13 is a schematic view for an explanatory description of a step of assessing transparency.
FIG. 14 is a schematic view showing results of an experimental example.

**Description of Embodiments**

Hereinafter, embodiments according to the present invention will be described with reference to the drawings. Identical elements are denoted by the same reference signs, and duplicate explanatory descriptions are omitted. The dimensional ratios in the drawings do not always coincide with those in the explanatory description.

In the present embodiment, an embodiment for assessing the safety of test substances will be described. The Ames test is known as a test for assessing the safety of test substances.

In the Ames test, a cell that has been subjected to genetic manipulation of an amino acid synthesis gene and thus has been modified so that the amino acid synthesis is not possible is used as a tester. In the Ames test, a test substance is added to the above-described cell. After that, the cell is cultured under certain conditions. In a case where the test substance causes a mutation of an amino acid synthesis gene, the cell can synthesize the amino acid again. As a result, genotoxicity is assessed by checking whether or not a mutation has occurred by checking the presence or absence of cell proliferation.

In the Ames test, a toxicity assessment for checking whether or not a test substance is toxic to cells may also be carried out. A transparency assessment method according to the present embodiment is suitably used to assess whether or not such a test substance is toxic to cells.

Hereinafter, a case where the transparency assessment method according to the present embodiment is applied to the Ames test will be described as an example. In the following explanatory description of the embodiment based on the Ames test, the tester is a cell unless otherwise specified.

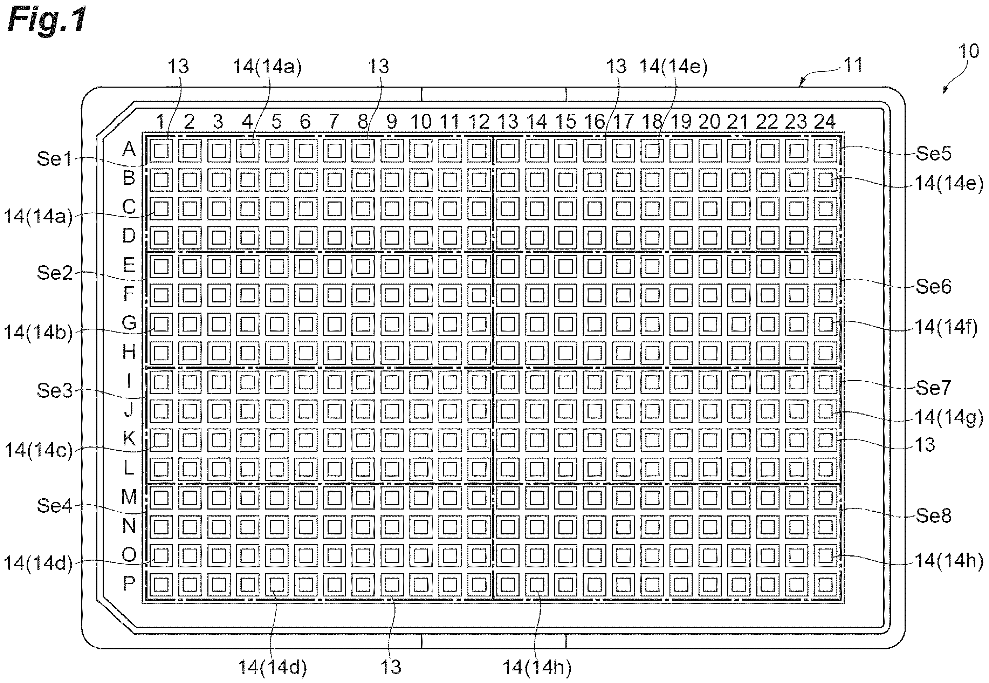

FIG. 1 is a plan view of an assessment plate 10 that is used in a transparency assessment method according to one embodiment. The assessment plate 10 has a plate 11. The plate 11 has a plurality of wells 13. In the present embodiment, the plate 11 has wells 13 of 384 wells arranged in two dimensions (16 × 24).

In the plate 11, N sections (predetermined regions) are virtually set. N represents an integer of 1 or more. In the present embodiment, as shown in FIG. 1, a case where eight sections are set virtually (that is, a case where N is 8) will be described. The eight sections are referred to as sections Se1 to Se8, respectively. The sections Se1 to Se8 are a region that includes the wells 13 of $4 \times 12$ (48) wells (well group). In the present embodiment, the section Se1 is a solvent control section (a region in which a reference target color appears), and the section Se8 is a positive control section (a region in which a comparative control color appears). The solvent control section functions as a negative control section.

The well 13 is a recessed part (depression) that is formed in the plate 11. The well 13 holds an assessment target 14. In a case where the assessment targets 14 held in the wells 13 belonging to the respective sections Se1 to Se8 are described separately, they are referred to as assessment targets 14a to 14h, respectively.

The assessment targets 14a to 14h have a culture solution in which cells are dispersed (or suspended). All of the wells 13 of 384 wells hold the same amount of culture solution. The quantity of cells in the culture solution held in the well 13 is also the same between the wells 13. Examples of cells include a microorganism having an amino acid-requiring mutation and a cell derived from a living organism. The microorganism having an amino acid-requiring mutation includes histidine-requiring Salmonella typhimurium (for example, TA1535, TA1537, TA97, TA97a, TA98, TA100, TA92, TA94, TA102, TA104, TA1538, TA7001, TA7002, TA7003, TA7004, TA7005, TA7006, various YG strain, and the like), tryptophan-requiring Escherichia coli (for example, WP2, WP2uvrA, WP2/pKM101, WP2uvrA/pKM101, and the like), other microorganisms, and the like. Examples of the cell derived from a living organism include cultured cells derived from mammals, cells derived from vertebrates other than the mammals, cells derived from insects, and the like. The cells included in the assessment targets 14a to 14h may be cells in which a plurality of kinds of cells are mixed. Examples of the culture solution include a phosphate buffer solution containing a small amount of amino acids.

The assessment targets 14a to 14h further contains an indicator. The amount of indicator is the same in the assessment targets 14a to 14h (that is, the same in all the wells 13). In the present embodiment, the indicator is a pH indicator. Examples of the pH indicator include bromocresol purple. The color of the pH indicator in the present embodiment is purple (target color) in a case of a negative result (in a case where no mutation occurs in the cell) or yellow (comparative control color) in a case of a positive result (in a case where mutation occurs in the cell).

The assessment targets 14b to 14g further include a solution (test substance solution) in which a test substance is dissolved or uniformly dispersed in a solvent in addition to the culture solution in which the above-described cells are dispersed, and an indicator. Accordingly, the wells 13 in the sections Se2 to Se7, which respectively hold the assessment targets 14b to 14g, are wells (test substance wells) holding the test substance. The test substance is a substance (for example, a chemical substance) for which safety (for example, cytotoxicity or genotoxicity) should be assessed. Examples of the solvent include water, dimethyl sulfoxide (DMSO), and the like. The assessment targets 14b to 14g are the same except that the concentration, which is one of the test conditions of the test substance, is different. The amount of test substance solution in the assessment target 14b in all the wells 13 belonging to the section Se2 is the same. The same applies to the sections Se3 to sections Se7.

The assessment target 14a contains a culture solution containing the above-described cells, an indicator as well as a solvent control solution that substantially does not affect the color of the indicator and cells. As a result, the well 13 in the section Se1, which holds the assessment target 14a, is a solvent control well (first reference target color well). The solvent control solution is a solvent used for the test substance solution. However, the solvent control solution may be any liquid that does not affect cells. Examples of the solvent control solution include water and DMSO. In all the wells 13 belonging to the section Se1, the amount of the solvent control solution in the assessment target 14a is the same.

The assessment target 14h contains a culture solution containing the above-described cells, an indicator as well as a positive control solution that changes the color of the indicator to yellow. As a result, the well 13 in the section Se8, which holds the assessment target 14h, is a positive control well (comparative control color well). The positive control solution is a solution containing a positive control compound and a solvent. The positive control compound may be a compound that is known as positive with respect to cells. Examples of the positive control compound include 9AA (9-aminoacridine), 4-NQO (4-nitroquinoline-N-oxide), 2-AA (2-aminoanthracene), and 2-NF (2-nitrofluorene). The solvent may not be the same as the solvent used for the test substance solution. Examples of the solvent include water, DMSO, and the like. The amount of positive control solution held in all the wells 13 belonging to the section Se8 which is a positive section is the same.

For example, in the Ames test, in a case of investigating the effect of the presence or absence of metabolic activation of the test substance, the assessment targets 14a to 14h further contain an S9 mix. The S9 mix is a liquid obtained by adding a coenzyme to an extract of animal liver. The S9 mix may be a liquid obtained by adding a cofactor involved in the electron transport system such as NADPH to a $9,000 \times g$ supernatant (S9) of the liver homogenate.

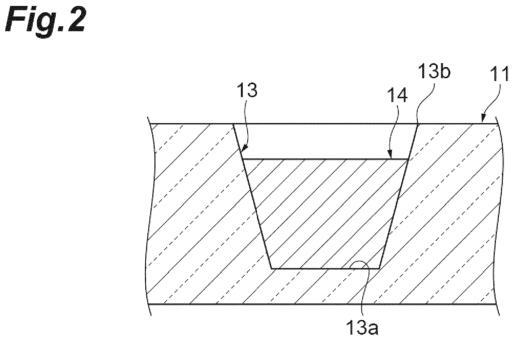

With reference to FIG. 1 and FIG. 2, the configuration of the well 13 will be further described. FIG. 2 is a schematic view of a cross section of a well. FIG. 2 schematically illustrates the assessment target 14. In the present embodiment, the shape of the well 13 in a plan view (the shape viewed in a depth direction of the well 13) is a square shape. An example of the length of one side of the opening portion of the well 13 is 3.0 mm to 4.0 mm. An example of the depth of the well 13 is 9.0 mm to 15.0 mm. The plate 11 having wells having such a size is known as a microplate. As shown in FIG. 2, the well 13 tapers toward a bottom part 13a. As a result, the area of the bottom part 13a is smaller than the area of an opening 13b of the well 13. The shape of the well 13 in a plan view may be a circle shape. In a case where the shape of the well 13 in a plan view has corners as in the case of the square, the corners may be rounded. The well 13 may have a column shape that does not taper toward the bottom part 13a.

The assessment plate 10 is a plate that enters a state in which the safety assessment for the assessment target 14 becomes possible after a corresponding assessment target 14 is injected into each of the wells 13 of the plate 11 and a certain culture treatment is performed.

The preparation procedure for the assessment plate 10 will be described based on an example of the Ames test. The assessment plate 10 may be prepared, for example, by the following procedure.

(Step 1A)

A tester (bacterium having amino acid-requiring mutation, for example, Salmonella typhimurium: a TA100 strain, a TA98 strain, a TA1535 strain, or a TA1537 strain, Escherichia coli: a WP2uvrA strain or the like) that is used is inoculated in 60 mL of Nutrient broth medium, and is shaken and cultured at 37°C for 9 to 10 hours to be proliferated.

(Step 2A)

A required amount of a test substance (for example, a compound of which toxicity is desired to be assessed) is weighed.

(Step 3A)

The test substance prepared in the step 2A is dissolved or suspended in an adequate medium, and the resultant solution is diluted by using the medium in staged to prepare concentration series of the test substance solution. In a case where the same test substance is assessed with six concentrations different from each other, six test substance solutions having concentrations different from each other are prepared.

(Step 4A)

A solution of a positive control compound, which is known to exhibit a predetermined toxicity, is prepared.

(Step 5A)

The medium used in preparation of the test substance solution, the concentration series of the test substance solution, and the solution of the positive control compound (collectively referred to as "test substance solution or the like") are put into individual wells of a 24-well plate (a plate having 24 wells) by 10 μL.

(Step 6A)

0.175 to 0.7 mL of the tester culture solution (typically at a concentration exceeding $1 \times 10^9$ cells/mL) and 6.83 to 6.3 mL of a medium (minimal medium in which a carbon source is added and which is deficient in amino acid) are mixed. The resultant mixture is referred to as a preparation bacterial solution under conditions of the absence of metabolic activation.

(Step 7A)

240 μL of preparation bacterial solution under conditions of the absence of metabolic activation prepared in the step 6A is added to the 24-well plate into which 10 μL of the test substance solution or the like has been put.

(Step 8A)

The 24-well plate into which the mixed solution of the step 7A is put is shaken at 37°C at 250 rpm for 90 minutes (pre-incubation).

(Step 9A)

2.6 mL of an indicator medium (a minimal medium which contains a pH indicator and a carbon source and which is deficient in amino acid) is added to each well (250 μL) of the 24-well plate after the termination of the pre-incubation.

(Step 10A)

The mixed solution in the step 9A is transferred to 48 wells (corresponding to one section) provided in each of the eight sections Se1 to Se8 of the plate 11 that is a 384-well plate by 50 μL so as to correspond to the sections Se1 to Se8. In the same manner, the mixed solution is seeded to three or more 384-well plates (plates 11).

(Step 11A)

The 384-well plates (plates 11) are cultured at 37°C for 48 hours. According to this, the assessment plate 10 is obtained.

In a case of detecting a test substance that exhibits toxicity after being metabolism by various enzymes in the living body, in the step 6A, as described above, a solution (S9 mixture) obtained by adding a coenzyme to an extract of animal liver is prepared, and 0.175 to 0.7 mL of tester culture solution, 5.78 to 5.25 mL of medium, and 1.05 mL of the S9 mixture are mixed. The resultant mixture is referred to as a prepared bacterial solution under the conditions of the presence of metabolic activation. Thereafter, the steps after the step 7A are carried out using the preparation bacterial solution under conditions of the presence of metabolic activation instead of the preparation bacterial solution under conditions of the absence of metabolic activation.

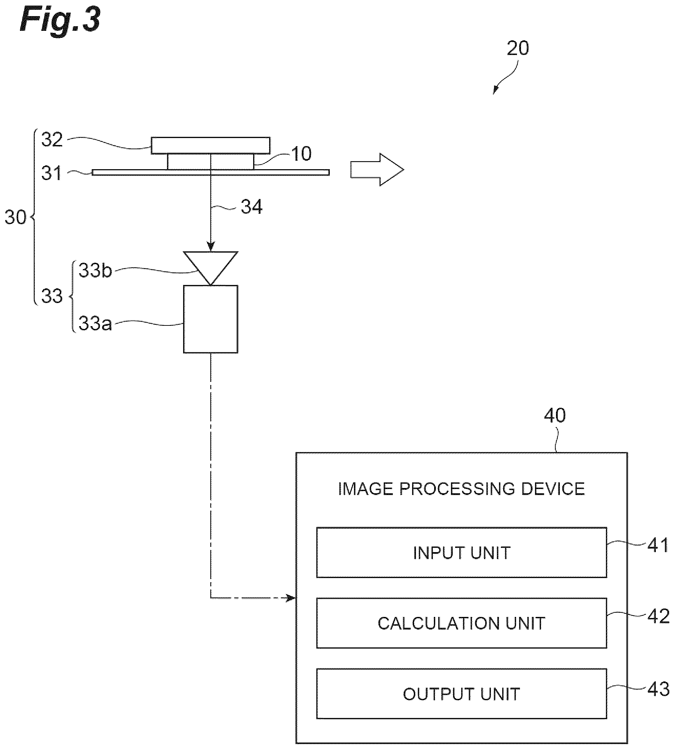

Next, an assessment system 20 that is used for the assessment method for the safety of the test substance held in the assessment plate 10 will be described. FIG. 3 is a schematic view of the assessment system 20 according to one embodiment. The assessment system 20 includes an observation device 30 and an image processing device 40.

[Observation device]

The observation device 30 includes a stage (plate holding unit) 31, a lighting device 32, and an imaging unit 33. The stage 31 supports the assessment plate 10. The assessment plate 10 is disposed on the stage 31 so that the bottom part of the assessment plate 10 (on the bottom part 13a side of the well 13) is located on the stage 31 side. The stage 31 is configured so that the light 34 output from the lighting device 32 passes toward the imaging unit 33. For example, a material of the stage 31 may have light transmittance, or an opening or a window part for transmitting the light 34 may be formed in a region in the assessment plate 10, which corresponds to the region in which the wells 13 of 384 wells are formed. In the present embodiment, the stage 31 is configured to be conveyed in the direction of the white blank arrow in FIG. 3.

The lighting device 32 is disposed on the assessment plate 10 (on a side opposite to the stage 31 in a case of being viewed from the assessment plate 10). The lighting device 32 irradiates the assessment plate 10 with the light 34. An example of the lighting device 32 is an LED lighting, and an example of the light 34 is light having a wavelength of 400 nm to 780 nm.

The imaging unit 33 receives the light 34 transmitted through the assessment plate 10, and images of the assessment plate 10. The imaging unit 33 acquires a color image (RGB image) that is substantially the same as the image in a case where the assessment plate 10 is visually observed. The imaging unit 33 includes an imager 33a and a light concentrator 33b.

Examples of the imager 33a include a two-dimensional sensor (or camera) and a line sensor. Examples of the two-dimensional sensor include a CCD camera and a CMOS camera.

The light concentrator 33b concentrates the light 34 and allows the concentrated light to be incident on the imager 33a. The light concentrator 33b includes at least one lens. In a case where the imager 33a itself has an optical function for light concentration, the imaging unit 33 may not have the light concentrator 33b.

In the present embodiment, the imaging region of the imaging unit 33 is smaller than the size of the assessment plate 10. In this case, the imaging unit 33 images the assessment plate 10 a plurality of times. However, the imaging unit 33 may include a plurality of imaging units 33, and the imaging region of the imaging unit 33 may be a size capable of imaging the entirely of the assessment plate 10 at one time.

The imaging unit 33 is connected to the image processing device 40 by wired communication or wireless communication and outputs the acquired image (specifically, image data) to the image processing device 40. The input of the image from the imaging unit 33 to the image processing device 40 may be carried out, for example, via a storage medium such as a DVD.

In the observation device 30, the positions of the lighting device 32 and the imaging unit 33 with respect to the assessment plate 10 may be inverted. That is, in FIG. 3, the assessment plate 10 may be imaged in a state in which the imaging unit 33 is disposed on the assessment plate 10 and the lighting device 32 is disposed on the lower side of the stage 31.

A method of imaging the assessment plate 10 using the observation device 30 will be described. In a case of imaging he assessment plate 10, the assessment plate 10 is disposed on stage 31. While moving the stage 31 in one direction, the assessment plate 10 is irradiated with the light 34 from the lighting device 32 (irradiation step). In a state in which the assessment plate 10 is irradiated with the light 34, the imaging unit 33 images the assessment plate 10 (imaging step).

In the present embodiment, the imaging region of the imaging unit 33 is smaller than the size of the assessment plate 10. Therefore, a partial image of the assessment plate 10 is obtained with one time of imaging. Therefore, in the imaging step, the assessment plate 10 is imaged a plurality of times using the imaging unit 33 so that the entire image of the assessment plate 10 is formed by composing the partial images.

The imaging unit 33 inputs the acquired image to the image processing device 40.

[Image processing device]

The image processing device 40 includes an input unit 41, a calculation unit 42 (execution unit), and an output unit 43. The image processing device 40 is a computer. In general, the image processing device 40 may further include a storage unit (memory) or the like possessed by a computer. The image processing device 40 may be a personal computer that functions as the image processing device 40 by executing various programs, or it may be a dedicated device for assessment. The image processing device 40 functions as an assessment device or an assessment support device for assessing the assessment plate 10.

The input unit 41 is an input means to which data of an image acquired by the imaging unit 33 are input (or received).

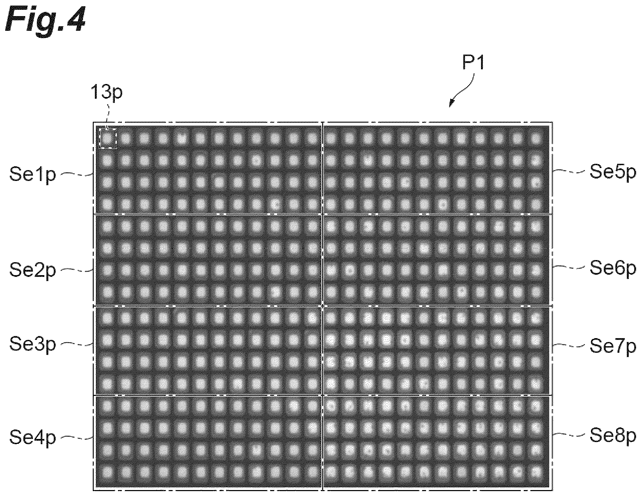

The calculation unit 42 creates a plate image P1 (see FIG. 4) based on the image input to the input unit 41. FIG. 4 is a drawing showing an example of the plate image P1. The plate image P1 is an image corresponding to the assessment plate 10. In the plate image P1, the portions corresponding to the well 13 and the section Se1 to Se8 are a well image 13p and section images Se1p to Se8p, respectively. The calculation unit 42 executes various processes for carrying out each step of the transparency assessment method according to the present embodiment, which will be described later. The calculation unit 42 executes each step by, for example, executing a program that allows a computer to execute each step of the transparency assessment method. Such a program may be stored, for example, in the storage unit (memory).

In a case where the input unit 41 outputs, for example, the plate image P1 to the storage unit, the calculation unit 42 needs only to access the storage unit and then acquire data to be processed. The calculation unit 42 will be described later.

The output unit 43 carries out display output so that the results of the calculation unit 42 are referenced by an assessor. The output unit 43 may output the results of the calculation unit 42 to, for example, an external display device, or the output unit 43 itself may have a display function. The output unit 43 may output the display of data during the processing process in the calculation unit 42.

Next, an example of the transparency assessment method according to the present embodiment will be described. Here, it is assessed whether or not a test substance is toxic to cells (tester). When the Ames test is carried out, if a test substance is toxic to cells (tester), the transparency of the well 13 (specifically, the transparency of the assessment target 14) changes due to the death of the cells or the like. In the present embodiment, a case of assessing the change in transparency will be mainly described.

As described above, in the present embodiment, a pH indicator that changes from purple to yellow in a case where the test substance is genotoxic to cells is used. Therefore, the color of the well 13 is purple in a case where the test substance is not genotoxic to the cell. Therefore, a form in which the transparency of purple of the sections Se1 to Se8 is assessed will be described. The above-described "the color of the well 13" is the color of the assessment target 14 held in the well 13.

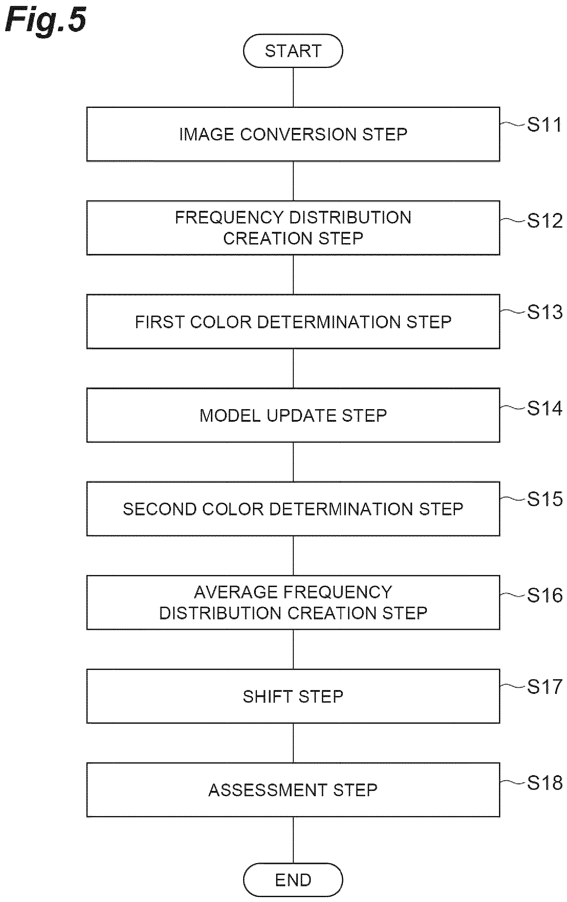

Hereinafter, a step after the image data of the assessment plate 10 has been acquired using the imaging unit 33 shown in FIG. 3, and the image data has been input to the image processing device 40 will be described with reference to FIG. 5.

In a case where the image data are input to the image processing device 40, the image processing device 40 converts the input image of the plate (color image, RGB image) into an HSV image expressed by a combination of three of "hue", "saturation", and "value" (an image conversion step S11). The conversion from the RGB image into the HSV image needs only to be carried out based on a publicly known conversion expression (or a publicly known program based thereon).

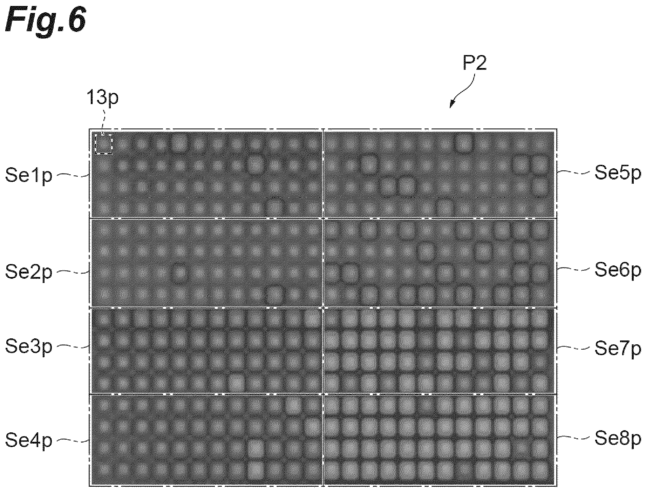

FIG. 6 is a drawing showing an example of an HSV image P2. The HSV image P2 shown in FIG. 6 is an image obtained by subjecting the plate image P1 shown in FIG. 4 to the HSV conversion. Since the HSV image P2 is an image obtained by subjecting the plate image P1 to the HSV conversion, in FIG. 6, the partial images corresponding to the well 13 and the sections Se1 to Se8 in the HSV image P2 are also illustrated as the well image 13p and the section images Se1p to Se8p, respectively.

Next, as shown in FIG. 5, the created HSV image P2 is used to create a frequency distribution (initial frequency distribution) having at least two color feature quantities as variates for each of the plurality of well images 13p (a plurality of regions) (a frequency distribution creation step S12). In the following explanatory description of the present embodiment, hue and saturation are adopted as the at least two color feature quantities unless otherwise noted. That is, the frequency distribution in the following explanatory description is a frequency distribution having hue and saturation as variates.

An example of the method of creating a frequency distribution will be described. The image processing device 40 identifies a region of each well image 13p in the HSV image P2. For example, as shown by the broken line in FIG. 6, an ROI (region of interest) needs only to be set on the well image 13p. FIG. 6 shows a state in which an ROI is set on one well image 13p for exemplification. The ROI may be set as a region specified by an assessor. Alternatively, the image processing device may automatically set an ROI based on, for example, the data (shape, size, and the like) of the plate 11, which have been input in advance, the image of the plate 11 in a state in which nothing is placed in the well 13, and the luminance information of the HSV image.

The image processing device 40 creates a frequency distribution by using the image information (hue, saturation, and the like) of a plurality of pixels included in the specified well image 13p. In the present embodiment, a frequency distribution is created by dividing hue and saturation into 0 to 55 and then extracting a feature quantity (a combination of hue and saturation).

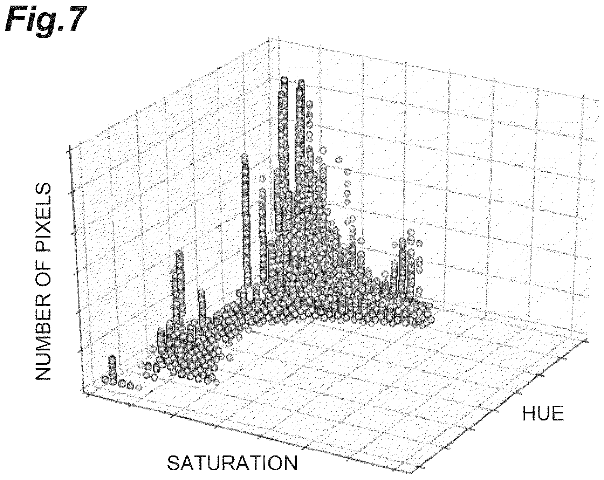

FIG. 7 is a drawing showing a histogram that is an example of the created frequency distribution. The ordinate of FIG. 7 indicates hue and saturation, and the abscissa indicates the number of pixels. FIG. 7 is a histogram for one well image 13p.

After the frequency distribution step S12, as shown in FIG. 5, the color of the well 13 included in each of the section Se1 and the section Se8 is determined (a first color determination step S13). The color of the well 13 is assumed to be purple and yellow, which are the colors before and after the color change of the pH indicator. Further, since the color appears to be different depending on the difference in the transparent state, purple is divided into purple in the first transparent state and purple in the second transparent state. As a result, in the first color determination step S13, it is determined what color the well 13 has among purple in the first transparent state, purple in the second transparent state, and yellow. Purple in the first transparent state is purple in the transparent state in a case where a test substance is not toxic to cells, and in the present embodiment, it is a color in a case where the transparency is low (there is turbidity). Purple in the second transparent state is purple in the transparent state in a case where a test substance is toxic to cells, and in the present embodiment, it is a color in a case where the transparency is high (there is no turbidity).

Hereinafter, purple in the first transparent state may be referred to as a first reference target color, and purple in the second transparent state may be referred to as a second reference target color.

An example of the first color determination step S13 will be specifically described.

In the first color determination step S13, first, the degree of similarity between a plurality of frequency distributions created in the frequency distribution creation step S12 and each of a frequency distribution model of the first reference target color (first frequency distribution model), a frequency distribution model of the second reference target color (second frequency distribution model), and a frequency distribution model (comparative control color frequency distribution model) of yellow is calculated.

Specifically, in a plurality of frequency distributions created in the frequency distribution creation step S12 and the frequency distribution model of each of the first reference target color, the second reference target color, and yellow, the degree of similarity of the first reference target color region, the second reference target color region, and the yellow region (comparative control color region) is calculated. The first reference target color region, the second reference target color region, and the yellow region are regions of each color specified by a combination of hue and saturation. For example, an assessor needs only to set the first reference target color region, the second reference target color region, and the yellow region in advance.

The degree of similarity may be calculated, for example, by using the Euclidean distance or may be calculated by using the correlation coefficient. The degree of similarity may be calculated, for example, by using pattern matching. Deep learning may be used to calculate the degree of similarity.

In the first color determination step S13, a well 13, which corresponds to a frequency distribution having a predetermined degree of similarity (for example, the maximum degree of similarity) with respect to the frequency distribution model of the first reference target color which has been calculated as described above, is determined to be the well 13 of the first reference target color.

In the same manner, a well 13, which corresponds to a frequency distribution having a predetermined degree of similarity (for example, the maximum degree of similarity) with respect to the frequency distribution model of the second reference target color, is determined to be the well 13 of the second reference target color.

In the same manner, a well 13, which corresponds to a frequency distribution having a predetermined degree of similarity (for example, the maximum degree of similarity) with respect to the frequency distribution model of yellow, is determined to be the well 13 of yellow.

The frequency distribution model of each of the first reference target color, the second reference target color, and yellow is a frequency distribution having hue and saturation as variates, and it is a frequency distribution serving as a reference for color determination. The frequency distribution model is, for example:

(a) it may be a frequency distribution created based on images of a plurality of wells exhibiting the first reference target color, the second reference target color, and yellow, by carrying out a preliminary experiment in advance,
(b) it may be a frequency distribution obtained for each color by specifying the first reference target color, the second reference target color, and yellow from the color palette list, or
(c) it may be a frequency distribution for regions, where the regions are specified to be obviously the first reference target color, the second reference target color, and yellow in the plate image P1.

After the first color determination step S13, as shown in FIG. 5, the frequency distribution of the well 13 which has been determined for each of the first reference target color, the second reference target color, and yellow in the first color determination step S13 is used to update the frequency distribution model of each of the first reference target color, the second reference target color, and yellow (a model update step S14).

Specifically, the frequency distribution of all the wells 13 which have been determined to be the first reference target color and the frequency distribution model for the first reference target color are additionally averaged to update the frequency distribution model for the first reference target color.

In the same manner, the frequency distribution of all the wells 13 which have been determined to be the second reference target color and the frequency distribution model for the second reference target color are additionally averaged to update the frequency distribution model for the second reference target color.

In the same manner, the frequency distribution of all the wells 13 which have been determined to be yellow and the frequency distribution model for yellow are additionally averaged to update the frequency distribution model for yellow.

The frequency distribution model that is used in steps after the model update step S14 is an updated frequency distribution model unless otherwise specified.

Next, the frequency distribution model of each of the first reference target color, the second reference target color, and yellow is used to determine the colors of all the wells 13 included in the assessment plate 10 (a second color determination step S15). The determination method is the same as in the case of the first color determination step S13, except that a frequency distribution corresponding to all the wells 13 included in the assessment plate 10 is used.

That is, a degree of similarity between all the frequency distributions created in the frequency distribution creation step S12 and the frequency distribution model of each of the first reference target color, the second reference target color, and yellow is calculated. A well 13, which corresponds to a frequency distribution having a predetermined degree of similarity (for example, the maximum degree of similarity) with respect to the frequency distribution model of the first reference target color, is determined to be the well 13 of the first reference target color.

In the same manner, a well 13, which corresponds to a frequency distribution having a predetermined degree of similarity (for example, the maximum degree of similarity) with respect to the frequency distribution model of the second reference target color, is determined to be the well 13 of the second reference target color.

In the same manner, a well 13, which corresponds to a frequency distribution having a predetermined degree of similarity (for example, the maximum degree of similarity) with respect to the frequency distribution model of yellow, is determined to be the well of yellow.

After the second color determination step S15, as shown in FIG. 5, an average frequency distribution of the first reference target color, the second reference target color, and yellow is created in each of the sections Se1 to Se8 (an average frequency distribution creation step S16).

Specifically, in each of the sections Se1 to Se8, the frequency distribution of the well 13 which has been determined to be the first reference target color is additionally averaged to create an average frequency distribution for the first reference target color of each of the sections Se1 to Se8.

In the same manner, in each of the sections Se1 to Se8, the frequency distribution of the well 13 which has been determined to be the second reference target color is additionally averaged to create an average frequency distribution for the second reference target color of each of the sections Se1 to Se8.

In the same manner, in each of the sections Se1 to Se8, the frequency distribution of the well 13 which has been determined to be yellow (comparative control color) is additionally averaged to create an average frequency distribution for yellow (comparative control color) of each of the sections Se1 to Se8.

Since an average frequency distribution for each color is created in this way in each of the sections Se1 to Se8, three average frequency distributions are obtained for each section.

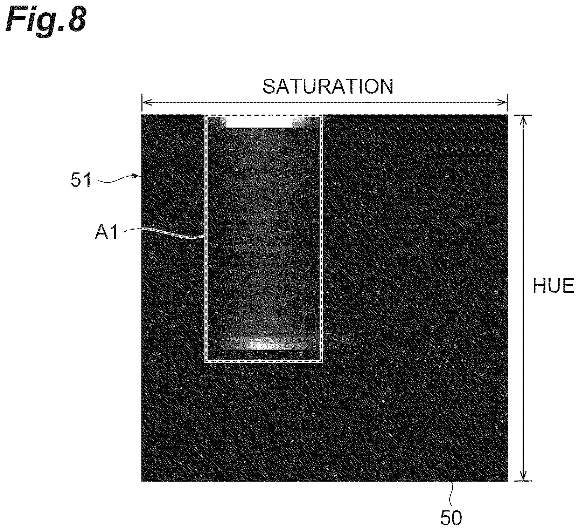

In the present embodiment, the transparency of purple (target color) of the sections Se1 to Se8 is assessed based on the average frequency distribution for each color, which has been created as described above for each of the sections Se1 to Se8. In the present embodiment, each average frequency distribution is used as an assessment image (image data for assessment). Specifically, the assessment image is an image (image data) obtained by expressing the frequency in the average frequency distribution as a luminance value and subjecting it to binarization processing. The assessment image in the present embodiment has the same size for all the average frequency distributions. The assessment image will be described with reference to FIG. 8 to FIG. 10. FIG. 8 to FIG. 10 are drawings for an explanatory description of an assessment image for each color, however, it is not an assessment image for each color in the same section.

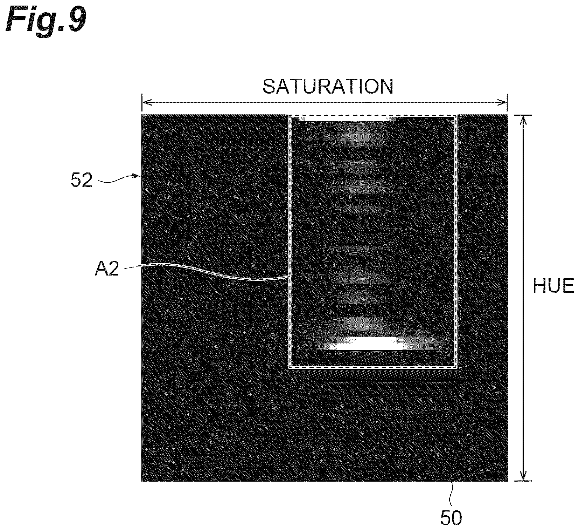

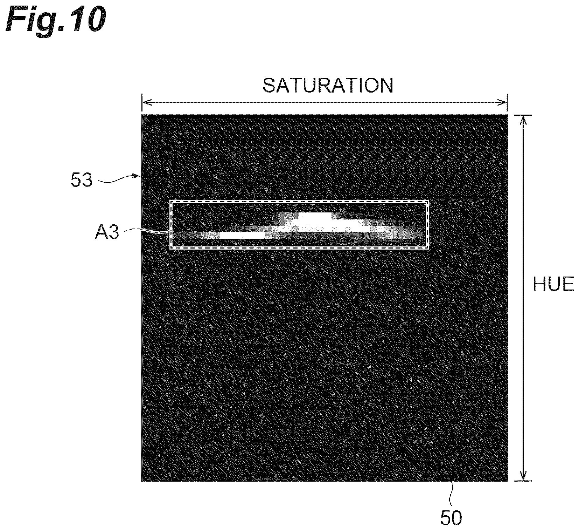

FIG. 8 is a drawing showing an example of an assessment image 51 which corresponds to the first reference target color. FIG. 9 is a drawing showing an example of an assessment image 52 which corresponds to the second reference target color. FIG. 10 is a drawing showing an example of an assessment image 53 which corresponds to yellow (comparative control color). The ordinate (horizontal direction) in each figure indicates saturation, and the abscissa (vertical direction) indicates hue. The white color region in each figure is a region having a high frequency (number of pixels) in the corresponding average frequency distribution.

Since FIG. 8 is an image corresponding to the first reference target color, the white color region is a first reference target color region A1 expressed by a combination of hue and saturation in an average frequency distribution of a section corresponding to the assessment image 51.

Similarly, since FIG. 9 is an image corresponding to the second reference target color, the white color region is a second reference target color region A2 expressed by a combination of hue and saturation in an average frequency distribution of a section corresponding to the assessment image 52.

Similarly, since FIG. 10 is an image corresponding to yellow (comparative control color), the white color region is a yellow region (comparative control color region) A3 expressed by a combination of hue and saturation in an average frequency distribution of a section corresponding to the assessment image 53. In FIG. 8 to FIG. 10, for the convenience of illustration, the first reference target color region, the second reference target color region, and the comparative control color region are shown by broken lines so that a plurality of white color regions are enclosed.

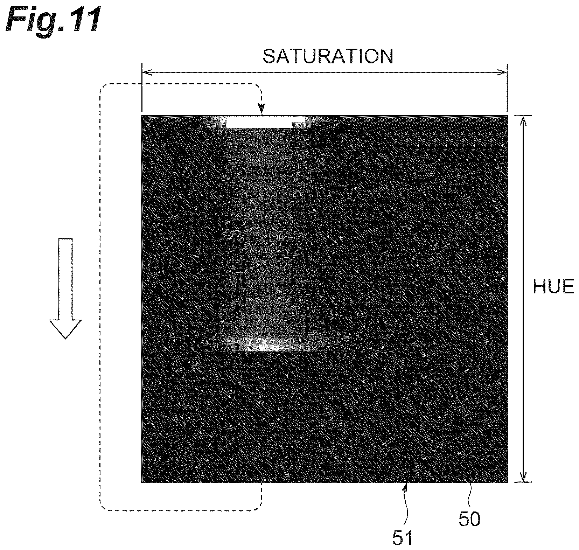

In the present assessment method, as shown in FIG. 5, after the average frequency distribution creation step S16, the first reference target color region A1, the second reference target color region A2, and the yellow region A3 in the images for assessments 51, 52, and 53 are shifted in a predetermined direction (a shift step S17). The shift step S17 will be described with reference to FIG. 11, by using, as an example, the case of the assessment image 51.

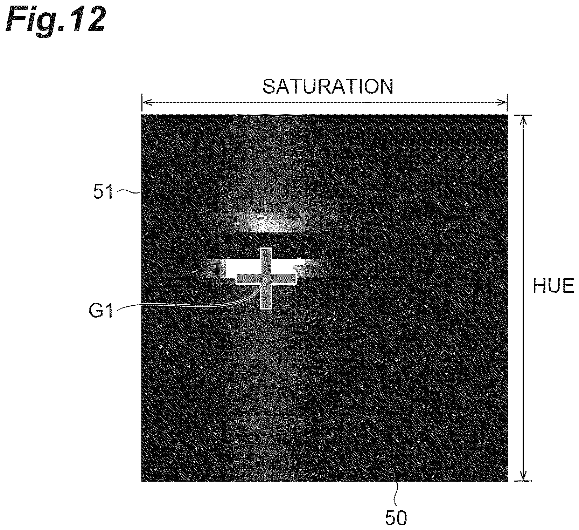

In the shift step S17, the assessment image 51 (specifically, the image data thereof) is shifted to a certain amount in the direction (predetermined direction) of the white blank arrow shown in FIG. 11. The example shown in FIG. 11 shows an example in which the image data are shifted in the hue direction. The image data of the assessment image 51 which moves out of the display region of the image due to the shift is moved (in other words, moved up) to a side opposite to the shift direction. For example, as shown in FIG. 11, in a case where the image data are shifted downward in the display region of the assessment image 51, the data drawn out from the display region is moved (moved up) to an edge (upper edge) side on a side (upper side) opposite to the display region as indicated by the broken line arrow. FIG. 12 is a drawing showing the assessment image 51 after the shift. In FIG. 12, the cross mark (specifically, the center position of the cross make) indicates a centroid position G1 of the shifted first reference target color region A1.

In the shift step S17, the image data are shifted in the same manner with respect to the assessment image 51 which has been created in each of the sections Se1 to Se8. The image data are shifted in the same manner with respect to the assessment image 52 and the assessment image 53, which have been created in each of the sections Se1 to Se8.

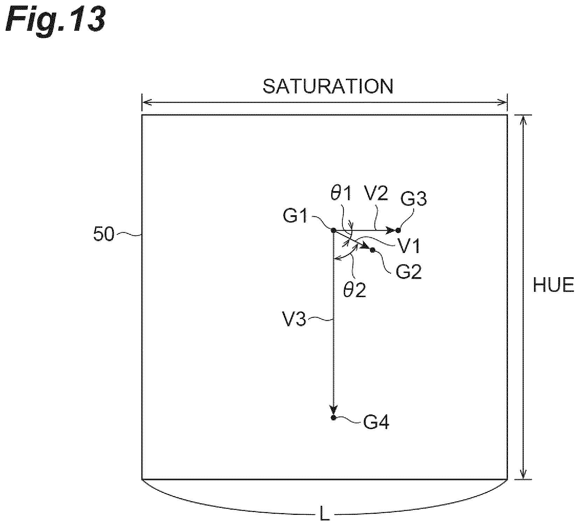

Next, after the shift step S17S, the transparency is assessed (determined) as shown in FIG. 5 (an assessment step S18 or determination step). The assessment step S18 will be described with reference to FIG. 13. FIG. 13 is a schematic view for an explanatory description of the assessment step S18.

In the assessment step S18, as shown in FIG. 13, a centroid vector V1, a first reference vector V2, and a second reference vector V3 are used to assess the transparency.

The centroid vector V1 is a vector directed from a reference centroid position (comparative original centroid position) for assessing transparency toward a centroid position of a transparency assessment region.

In the present embodiment, since the section Se1 is a solvent target section (negative control section) includes a solvent control well (first reference target color well) for the first reference target color, the transparency of the other sections is assessed using, as a reference, the transparency of purple of the section Se1. As a result, in the present embodiment, the centroid position (first centroid position) G1 of the first reference target color region A1 in the average frequency distribution (first reference frequency distribution) with respect to the first reference target color of the section Se1 is set to the reference centroid position.

Since the transparency of purple (target color) in the sections Se1 to Se8 is assessed, the transparency assessment region is the purple region (target color region). In the present embodiment, in the sections Se1 to Se8, a region obtained by combining the first reference target color region A1 of the average frequency distribution for the first reference target color (purple in the first transparent state) and the second reference target color region A2 of the average frequency distribution for the second reference target color (purple in the second transparent state) is adopted as the transparency assessment region.

Such a transparency assessment region (target color region) can be acquired, for example, by the following first method or second method.

[First method]

First, the average frequency distribution for the first reference target color (purple in the first transparent state) and the average frequency distribution for the second reference target color (purple in the second transparent state), which have been created in the average frequency distribution creation step S16, are additionally averaged to create an average frequency distribution of purple (target color) in each of the sections Se1 to Se8 (a step of acquiring N frequency distributions). The image data in a case where the average frequency distribution is displayed as an image is subjected to the shift step S17. A transparency assessment region is set based on an image corresponding to the average frequency distribution of purple which has been obtained through the shift step S17.

[Second method]

The transparency assessment region may be set in an image obtained by superimposing the images respectively corresponding to the average frequency distribution for the first reference target color (purple in the first transparent state) and the average frequency distribution for the second reference target color (purple in the second transparent state) after going through the shift step S17. Superimposing the images corresponding to the average frequency distribution for the first reference target color (purple in the first transparent state) and the average frequency distribution for the second reference target color (purple in the second transparent state), respectively, corresponds to additionally averaging the average frequency distribution for the first reference target color (purple in the first transparent state) and the average frequency distribution for the second reference target color (purple in the second transparent state), thereby creating an average frequency distribution of purple (target color) (the step of acquiring N frequency distributions).

The centroid position of the transparency assessment region is referred to as the centroid position (second centroid position) G2. An example of the centroid position G2 of the transparency assessment region in each of the sections Se1 to Se8 will be described. The transparency assessment region is a purple region, and as described above, it is a region including the region of the first reference target color (the target color in the first transparent state) and the region of the second reference target color (the target color in the second transparent state). Such a transparency assessment region is acquired as described above in each of the sections Se1 to Se8, and the centroid position of the acquired transparency assessment region is defined as the centroid position G2.

As described above, in a case where the centroid position G2 of the transparency assessment region is defined from the centroid position G1 (reference centroid position), the centroid vector V1 is a vector directed from the centroid position G1 toward the centroid position G2 of each of the sections Se1 to Se8 as shown in FIG. 13.

The first reference vector V2 is a centroid vector directed from the centroid position G1 of the first reference target color region A1 toward the centroid position (third centroid position) G3 of the second reference target color region A2, and it is a transparency change reference vector indicating the direction of change in transparency in the same color (purple in the present embodiment).

The second reference vector V3 is a centroid vector directed from the centroid position G1 of the first reference target color region A1 toward the centroid position (fourth centroid position) G4 of the yellow region A3, and it is a color change reference vector indicating the direction of color change from purple to yellow.

Here, as described above, since the centroid position G1 of the first reference target color region A1 of the section Se1 is used as the base point of the centroid vector V1, the centroid position G1 of the first reference target color region A1 of the section Se1 is also adopted for the centroid position G1 of the first reference target color region A1, which is the base point of the first reference vector and the second reference vector.

Regarding the centroid position G3 of the second reference target color region A2, the centroid position G3 of the second reference target color region A2, which is included in the average frequency distribution for the second reference target color in any of the sections Se1 to Se8, can be adopted. For example, the centroid position of the second reference target color region A2 in the average frequency distribution (second reference frequency distribution) with respect to the second reference target color in the section Se7 can be adopted. This is because the color of the well 13 included in the section Se7 is likely to be the second reference target color in a case where the test substance is tentatively toxic to cells. In the present embodiment, as described about the first reference target color region A1 with reference to FIG. 12, the centroid position of the second reference target color region A2 that has undergone the shift step S17 is adopted as the centroid position G3.

Similarly, regarding the centroid position G4 of the yellow region A3, the centroid position G4 of the yellow region A3 included in the average frequency distribution for the yellow region in any of the sections Se1 to Se8 can be adopted. For example, the centroid position G4 of the yellow region A3 of the average frequency distribution in the section Se8 can be adopted. Since the section Se8 is a positive control section and is assumed to change to yellow, a more accurate centroid position G4 can be obtained as the centroid position of the yellow region A3 by using the yellow region A3 of the average frequency distribution (comparative control frequency distribution) with respect to yellow in the section Se8. In the present embodiment, as described about the first reference target color region A1 with reference to FIG. 12, the centroid position of the yellow region A3 undergoing the shift step S17 is adopted as the centroid position G4.

In the assessment step S18, transparency is assessed using the centroid vector V1, the first reference vector V2, and the second reference vector V3.

Specifically, first, an angle $\theta 1$ between the centroid vector V1 and the first reference vector V2 and an angle $\theta 2$ between the centroid vector V1 and the second reference vector V3 are compared. In a case where the angle $\theta 1$ is equal to or smaller than the angle $\theta 2$, a transparency index T is calculated based on an expression (1). In a case where the angle $\Theta 2$ is larger than the angle $\theta 1$, the transparency index T is calculated based on an expression (2).

$$T = 1 - \mathrm{Ln}\,(\theta 1 \leq \theta 2)\,\cdots\,(1)$$

$$T = 1 + \mathrm{Ln}\,(\theta 1 > \theta 2)\,\cdots\,(2)$$

"Ln" in the expressions (1) and (2) is a length obtained by dividing the length of the centroid vector V1 by the image size (length of one side L) of the assessment image, and it is a normalized length (or normalized length) of the centroid vector V1.

Based on the assessment method for the assessment step S18, the fact that the transparency index T is smaller than 1 means that the transparency is high (in the present embodiment, it means that cells are dead).

That is, it indicates that the smaller than 1 the transparency index T is, the more toxic to cells a test substance is. This point will be described in detail.

A case where the transparency index T is less than 1 is the case of the expression (1), that is, a case where the angle θ1 is equal to or smaller than the angle Θ2. In this case, the centroid vector V1 is located on the first reference vector V2 side. Further, in the expression (1), the larger the length Ln of the standardized centroid vector V1 is, the smaller T is. The fact that the length Ln of the standardized centroid vector V1 is large means that the centroid position G2 is far from the centroid position G1. Therefore, in a case where the transparency index T is less than 1, it indicates that the centroid position G2 is located from the centroid position G1 to the centroid position G3 side, and as a result, it means that the degree of transparency is high. As a result, it is possible to objectively and quantitatively assess the transparency by using the transparency index T. For example, in a case where a certain threshold value is determined in advance for the transparency index T and the transparency index T is smaller than the threshold value, a test substance can be assessed to have toxicity to cells.

The transparency index T is calculated for each of the sections Se 1 to Se8. Therefore, transparency, that is, toxicity, can be assessed for each of the sections Se1 to Se8. Alternatively, for example, in a case where the same test substance is used except for the difference in concentration in the sections Se1 to Se8, which is a case where the transparency index T in any of sections Se1 to Se8 is smaller than the above threshold value, the test substance may be determined to be toxic to cells from the viewpoint of safety.

In the present transparency assessment method, it is possible to assess substantially automatically the transparency of the sections Se1 to Se8 in the assessment plate 10 by analyzing the plate image P1. Therefore, the objectivity and accuracy of the transparency assessment can be improved, for example, as compared with a case where an assessor carries out assessment visually. Further, since it is possible to assess substantially automatically the transparency of the sections Se1 to Se8 in the assessment plate 10 by analyzing the plate image P1, the reproducibility of the assessment is also improved.

Since the centroid position G1 of the first reference target color region A1 corresponding to purple of the first transparent state is adopted as the assessment standard, The transparency change can be assessed with high accuracy, for example, as compared with a case where the purple region including the first transparent state and the second transparent state is used as a reference.

Since the transparency can be assessed objectively and with higher accuracy as described above, the toxicity of the test substance to the cells can also be assessed more accurately based on the assessment results of the transparency of each of the sections Se1 to Se8.

As one embodiment, a case having the model update step S14 will be described. It is possible to obtain a frequency distribution model of each color according to the assessment plate 10 by updating, in the model update step S14, the frequency distribution model of each color based on the assessment plate 10 to be assessed. As a result, the transparency of the sections Se1 to Se8 in the assessment plate 10 can be more appropriately assessed, and as a result, the accuracy of the toxicity assessment of the test substance on the cells is improved.

The centroid positions G1, G2, G3, and G4, which define the centroid vector V1, the first reference vector V2, and the second reference vector V3, are also acquired based on an image obtained by imaging the assessment plate 10. Therefore, the centroid vector V1, the first reference vector V2, and the second reference vector V3 according to the assessment plate 10 can be defined as compared with, for example, a case where the centroid positions G1, G2, G3, and G4 which are obtained with respect to the initial frequency distribution model of each color. As a result, the transparency of the sections Se1 to Se8 in the assessment plate 10 can be more appropriately assessed, and as a result, the accuracy of the toxicity assessment of the test substance on the cells is improved.

In a case where the assessment method includes the shift step S17, it is easy to set the second reference vector V3. This is because in a case where the shift step S17 is not provided, a case where a sufficient distance cannot be secured between the centroid position G1 of the first reference target color region A1 and the centroid position G4 of the yellow region A3 may occur. Even in such a case, the centroid position G1 of the first reference target color region A1 and the centroid position G4 of the yellow region A3 can be appropriately separated by carrying out the shift step S17. Thus, since the shift step S18 is a step that is carried out to appropriately separate the centroid position G1 of the first reference target color region A1 and the centroid position G4 of the yellow region A3, the shift amount, shift direction, and the like need only to be set so that the centroid position G1 of the first reference target color region A1 and the centroid position G4 of the yellow region A3 are separable.

In the present embodiment, a case in which an example of the assessment method according to the present embodiment is applied to the Ames test has been described. In the above-described embodiment, an average frequency distribution of the first reference target color, the second reference target color, and yellow is created in each of the sections Se1 to Se8 in the average frequency distribution creation step S16. The average frequency distribution of purple (target color) can be obtained using the average frequency distribution of the first reference target color and the second reference target color. A frequency distribution model of purple (target color) can also be created based on the frequency distribution model of each of the first reference target color and the second reference target color. As a result, by calculating a degree of similarity between the average frequency distribution of each of purple and yellow of the sections Se1 to Se8 and the frequency distribution model of each of purple and yellow, it is possible to determine whether the color of the sections Se1 to Se8 is purple or yellow. As a result, genotoxicity can also be assessed based on the results of color determination.

In the present embodiment, since an image of the assessment plate 10 is acquired, the presence or absence of a colony in the well 13 can be determined or the presence or absence of a foreign substance can be determined by image processing. The presence or absence of genotoxicity can also be assessed by the presence or absence of the colony. The foreign substance is a foreign substance that is caused, for example, by the death of cells under the influence of the test substance. Therefore, it is also possible to determine whether or not a test substance is toxic to cells by the presence or absence of a foreign substance. Such determination of the presence or absence of the colony, determination of the foreign substance, and the like may be carried out together with the assessment of genotoxicity by color determination in order to obtain more accurate assessment results in the Ames test. As a result, the assessment method according to the present embodiment, which can also carry out such assessment in parallel, is easy to be applied to the Ames test.

Next, an experimental example in which the assessment system 20 and the assessment method described in the present embodiment are applied to the Ames test will be described. Hereinafter, unless otherwise stated, the same reference numerals are assigned to elements that are the same as the elements described so far, and duplicate explanatory descriptions are omitted.

In the experimental example, View-Plate-384 manufactured by PerkinElmer, Inc. was used as the plate 11. The View-Plate-384 had the wells 13 of 384 wells as shown in FIG. 1. Even in the experimental example, as in FIG. 1, the sections Se1 to Se8 were set virtually.

In the experimental example, an Ames test kit ("Ames MPF™ Penta I Microplate Format Mutagenicity Assay") manufactured by Xenometrix, Inc., which contains an indicator, a culture solution, and the like, was used. The preparation procedure for the assessment plate 10 in the Ames test was carried out according to the procedure of the steps 1A to 11A described above.

The tester (cells), the test substance, the test substance solution and the positive control compound, and the positive control compound, which were used in the experimental examples, were as follows.

Tester (cell): TA1537
Test substance: 4-chlorobenzyl chloride
Solvent for test substance solution and positive control solution: DMSO

Positive control compound: 9AA

In the experimental example, the section Se1 was set as the negative control section, and the section Se8 was set as the positive control section. The concentrations of the test substance in the section Se2 to the section Se7 in the step 8A were as follows.

Section Se2: 1.95 $\mu$g/mL
Section Se3: 7.81 $\mu$g/mL
Section Se4: 31.3 $\mu$g/mL
Section Se5: 125 $\mu$g/mL
Section Se6: 500 $\mu$g/mL
Section Se7: 2,000 $\mu$g/mL

The concentration of the positive control compound in the section Se8 (positive control section) in the step 8A was 15 $\mu$g/mL. In the present experimental example, an experiment without metabolic activation was carried out.

Under the above-described conditions, the assessment plate 10 was prepared according to the procedure of the steps 1A to 11A described above, and the plate image P1 was obtained with the observation device 30 schematically shown in FIG. 3. A color camera having a resolution of 20 $\mu$m/pixel was used for the imaging unit 33 included in the observation device 30. The color camera was disposed on the lower side of the assessment plate 10 as shown in FIG. 3. An LED lighting was used for the lighting, and then light was irradiated from the upper side of the assessment plate 10. In the imaging of the assessment plate 10, the assessment plate 10 was imaged while conveying the assessment plate 10. The conveying speed was 4.28 mm/s.

The plate image P1 of the assessment plate 10, which had been obtained by the imaging as described above, was subjected to the transparency assessment method described with reference to FIG. 5. In the present experimental example, the transparency of each of the sections Se1 to Se7 was assessed. Since the section Se8 was a positive control section, the transparency index T of the section Se8 was not calculated. That is, the transparency of the section Se8 was not assessed. In the experimental example, the frequency distribution was a frequency distribution having hue and saturation as variates. Hue and saturation were assessed by dividing them into 0 to 55. For the frequency distribution model of the first color determination step S13, a frequency distribution for regions, where the regions are specified to be obviously the first reference target color, the second reference target color, and yellow in the plate image P1, was used. The maximum degree of similarity was adopted for the predetermined degree of similarity in the first color determination step S13 and the second color determination step S15. In the experimental example, the transparency assessment region (purple region) was set based on the first method.

In the present experimental example, three assessment plates 10 under the same conditions were prepared, and the transparency was assessed for the three assessment plates 10.

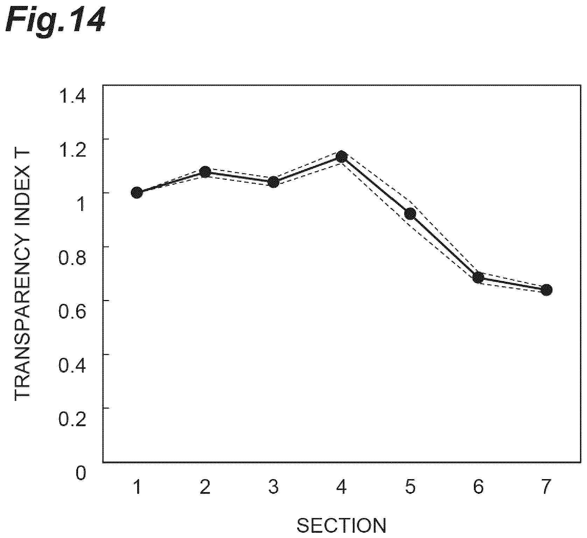

FIG. 14 shows the results obtained by plotting the transparency index T obtained according to the expression (1) and the expression (2) for each of the sections Se1 to Se7. The "1", "2", "···", and "7" on the ordinate of FIG. 14 indicate sections Se1, Se2, ···, and Se7, respectively. The abscissa is the transparency index T. Each plot point is the average value of the assessment results for the above-described three assessment plates 10. The broken line in the figure indicates a standard deviation from the assessment results for the above-described three assessment plates 10.

As shown in FIG. 14, the transparency index T is reduced in the section Se6 and the section Se7 which have a higher concentration of the test substance. In this case, it can be clearly understood that the test substance used is a test substance that is toxic to the tester. For example, by setting the threshold value slightly below 1 in advance, toxicity may be assessed by the presence or absence of a section in which the transparency index T is located below the threshold value.

As described above, the embodiment and the experimental example according to the present invention have been described. However, the present invention is not limited to the above-described embodiments and experimental examples, and the present invention is intended to include the scope indicated by the Claims and is intended to include all modifications within the meaning and the scope equivalent to the Claims.

In the above-described embodiment, a case in which the transparency assessment method according to the present invention is applied to the Ames test has been described. However, the safety assessment method to which the present transparency assessment method is applied is not limited to the Ames test, and it can also be applied to, for example, a test that uses a pH indicator, a test that uses a coloring agent (for example, an MTT assay or protein quantitation), and a test that uses luminescence or fluorescence (for example, an ATP assay).

In the above-described embodiment, since a form in which the present transparency assessment method is applied to the Ames test has been described, the embodiment has been described by using, as an example, a case where a color change from purple to yellow exists in the color of the indicator. However, the present transparency assessment method may be carried out focusing only on the change in the transparency of purple (target color).

In this case, it is possible to omit the acquisition or the like of a frequency distribution for yellow in the above-described embodiment. Since the frequency distribution for yellow is not used, it is also possible to omit the use of the second reference vector. In this case, the transparency needs only to be assessed based on the angle θ1 of the first reference vector and the centroid vector, and the length thereof. The transparency needs only to be assessed by, for example, setting a threshold value in advance for the angle θ1, and applying the expressions (1) and the expressions (2) according to the relationship between the threshold value and the angle θ1 instead of the angle Θ2. Since the shift step S17 in the above-described embodiment is a treatment for further separating the centroid position G1 of the first reference target color region A1 and the centroid position G4 of the yellow region A3, the shift step S17 may not be carried out in a case where the frequency distribution for yellow is not used.

In the above-described embodiment, the assessment was carried out using the angle θ1 and length of the centroid vector with respect to the first reference vector. However, since the vector has an orientation and size, transparency can be assessed in a case where the centroid vector is obtained.

In the above-described embodiment, transparency was assessed based on the relationship between the centroid vector and the first reference vector and the second reference vector. However, the first reference vector and the second reference vector may not be used. For example, in a frequency distribution having saturation and hue as variates, it is also possible to assess transparency based on the angle or the like of the centroid vector with respect to the direction of change in saturation (for example, the lateral direction of FIG. 8). In this case as well, a threshold value may be set as described above for the angle of the centroid vector with respect to the direction of change in saturation.

The average frequency distribution acquired for the sections Se1 to Se8 was used to specify the first reference target color region, the second reference target color region, and the yellow region. However, for example, the first reference target color region, the second reference target color region, and the yellow region in the frequency distribution model itself with respect to the first reference target color, the second reference target color, and yellow may be used.

The present transparency assessment method may not include the model update step S14 described in the above-described embodiment. In this case, in a treatment such as color determination using the updated frequency distribution model, an initial frequency distribution model (a frequency distribution model that is not updated) with respect to the first reference target color, the second reference target color, and yellow needs only to be used.

For the centroid position G3 of the second reference target color region A2, the centroid position of the second reference target color region, which is obtained by subjecting the second reference target color region in the frequency distribution model with respect to the second reference target color, to the shift step S17, may be used. It is noted that in a form in which the shift step S17 is not carried out, the centroid position of the second reference target color region in the frequency distribution model with respect to the second reference target color may be used. In these forms, the color determination with respect to the second reference target color in the first color determination step S13 may not be carried out, and in association with this, the frequency distribution model with respect to the second reference target color may not be subjected to the model update step S14. Such a form is particularly effective for example, in a case where a determination is made without dividing purple into a first reference target color and a second reference target color as a color determination of well 13 but using purple including the first reference target color and the second reference target color.

The first color determination step S13 and the model update step S14 may not be carried out. In this form, as a color determination step corresponding to the second color determination step S15, the color of each well 13 needs only to be determined based on the frequency distribution corresponding to each well 13, which has been created in the frequency distribution creation step S12. In one form in such a case, the color needs only to be determined based on the frequency in the first reference target color region, the second reference target color region, and the yellow region in the frequency distribution corresponding to each well 13. For example, the color of the well 13 corresponding to the frequency distribution, in which there is a frequency having a threshold value equal to or larger than a certain threshold value in the first reference target color region, needs only to be determined as the first reference target color. The color determination of the second reference target color and the color determination of yellow can be carried out in the same manner. In this case, the frequency distribution model corresponding to each of the first reference target color, the second reference target color, and yellow may not be used.

A case where the target color is purple and the comparative control color is yellow has been described. However, these colors correspond to the colors that serve as an indicator in the assessment test, and thus they are not limited to purple, yellow, and the like.

Instead of the frequency distribution having hue and saturation as variates, it is also possible to use a frequency distribution having, as variates, hue and brightness, or brightness and saturation. The number of variates defining the frequency distribution may be 3 or more.

The number (N) of predetermined regions in the plate image is not limited to 8. In the above-described embodiment, a case where the N predetermined regions in the plate image are regions corresponding to eight sections of the sections Se1 to Se8, which include the section Se1 (negative control section) and the section Se8 (positive control section), has been described. However, the N predetermined regions may be seven sections of the section Se1 to Se7, which do not include the section Se8 serving as the positive control section. In this case, the plate image may have a region corresponding to the positive control section (a region in which the comparative control color appears) together with the N predetermined regions. The N predetermined regions may be the sections Se2 to Se7, which do not include the section Se1 serving as the negative control section and the section Se8. In this case, together with the N predetermined regions, at least one of a region corresponding to the positive control section (a region in which the comparative control color appears) and a region corresponding to the negative control section (a region in which the first reference target color appears) may be provided.

The exemplified embodiments, modification examples, and the like may be appropriately combined without departing from the gist of the present invention.

Reference Signs List

10: Assessment plate, 13: Well, 14, and 14a to 14h: Assessment target, P1: Plate image, 13p: Well image, G1: Centroid position (first centroid position), G2: Centroid position (second centroid position), G3: Centroid position (third centroid position), G4: Centroid position (fourth centroid position), and Se1 to Se8: Sections (N predetermined regions).

**Claims**

1. A transparency assessment method comprising:

    a step of acquiring N frequency distributions (N is an integer of 1 or more) for a target color in N predetermined regions in a plate image, the N frequency distributions having at least two color feature quantities as variates; and
    a step of assessing transparency of the N predetermined regions based on a first reference frequency distribution for a first reference target color and the N frequency distributions, the first reference frequency distribution having the at least two color feature quantities as variates;
    wherein the plate image is an image corresponding to an assessment plate in which an assessment target is held in a plurality of wells provided in the plate,
    the assessment target includes a tester,
    the plurality of wells have at least one test substance well that holds the assessment target further including a test substance,
    at least one of the N predetermined regions includes at least one image corresponding to the at least one test substance well,
    the first reference target color is the target color that is in a first transparent state, and
    in the step of assessing the transparency, the transparency is assessed based on a centroid vector directed from a first centroid position of a first reference target color region in the first reference frequency distribution toward a second centroid position of a target color region in each of the N frequency distributions.

2. The transparency assessment method according to Claim 1, further comprising:

a step of acquiring the first reference frequency distribution,
wherein the plurality of wells further include at least one first reference target color well for the first reference target color,
one of the N predetermined regions includes an image corresponding to the at least one first reference target color well, and
in the step of acquiring the first reference frequency distribution, a frequency distribution having the at least two color feature quantities as variates in a predetermined region including the image corresponding to the at least one first reference target color well among the N predetermined regions is acquired as the first reference frequency distribution.

3. The transparency assessment method according to Claim 1 or 2, wherein in the step of assessing the transparency, the transparency is assessed based on an angle of the centroid vector with respect to a transparency change reference vector and a length of the centroid vector,

the transparency change reference vector is a vector directed from the first centroid position toward a third centroid position of a second reference target color region in a second reference frequency distribution for a second reference target color, the second reference frequency distribution having at least two color feature quantities as variates,
the second reference target color is the target color that is in a second transparent state different from the first transparent state, and
the length of the centroid vector is a length standardized by an image size in a case where a frequency distribution including the second centroid position defining the centroid vector is displayed as an image.

4. The transparency assessment method according to Claim 3, further comprising:

a step of acquiring the second reference frequency distribution,
wherein in the step of acquiring the second reference frequency distribution, a frequency distribution having the at least two color feature quantities as variates for the second reference target color in one predetermined region among the N predetermined regions is acquired as the second reference frequency distribution.

5. The transparency assessment method according to Claim 1, wherein in the step of acquiring the N frequency distributions, in each of the N predetermined regions, frequency distributions for a first reference target color and a second reference target color are acquired, and a frequency distribution of the target color is acquired based on the frequency distribution for the first reference target color and the frequency distribution for the second reference target color,

the second reference target color is the target color that is in a second transparent state different from the first transparent state,
in the step of assessing the transparency, the transparency is assessed based on an angle of the centroid vector with respect to a transparency change reference vector and a length of the centroid vector,
the transparency change reference vector is a vector directed from the first centroid position toward a third centroid position of a second reference target color region in a second reference frequency distribution, the second reference frequency distribution having at least two color feature quantities as variates,
the first reference frequency distribution is a frequency distribution for the first reference target color acquired in one predetermined region among the N predetermined regions,
the second reference frequency distribution is a frequency distribution for the second reference target color acquired in one predetermined region among the N predetermined regions, and
the length of the centroid vector is a length standardized by an image size in a case where a frequency distribution including the second centroid position defining the centroid vector is displayed as an image.

6. The transparency assessment method according to Claim 5, wherein the step of acquiring the N frequency distributions includes:

a step of acquiring an initial frequency distribution having the at least two color feature quantities as variates with respect to a plurality of regions in each of the N predetermined regions;
a step of determining colors of the plurality of regions based on a degree of similarity between the initial frequency distribution with respect to the plurality of regions and a first frequency distribution model for the first reference target color and a degree of similarity between the initial frequency distribution with respect to the plurality of regions and a second frequency distribution model for the second reference target color; and

a step of acquiring frequency distributions for the first reference target color and the second reference target color by using an initial frequency distribution of a region determined to correspond to the first reference target color and the second reference target color, respectively, among the plurality of regions.

7. The transparency assessment method according to Claim 1, further comprising:

a step of shifting image data corresponding to a frequency distribution having the at least two color feature quantities as variates in a direction of one of the at least two color feature quantities, wherein in the step of acquiring the N frequency distributions, in each of the N predetermined regions, frequency distributions for a first reference target color, a second reference target color, and a comparative control color are acquired, and concurrently, a frequency distribution of the target color is acquired based on the frequency distribution for the first reference target color and the frequency distribution for the second reference target color,

the second reference target color is the target color that is in a second transparent state different from the first transparent state,

the comparative control color is a color different from the target color,

in the step of assessing the transparency, the transparency is assessed based on an angle of the centroid vector with respect to a transparency change reference vector, an angle of a centroid vector with respect to a color change reference vector, and a length of the centroid vector,

the transparency change reference vector is a vector directed from the first centroid position toward a third centroid position of a second reference target color region in a second reference frequency distribution,

the color change reference vector is a vector directed from the first centroid position toward a fourth centroid position of a comparative control color region in a comparative control frequency distribution,

the first reference frequency distribution is a frequency distribution for the first reference target color acquired in one predetermined region among the N predetermined regions,

the second reference frequency distribution is a frequency distribution for the second reference target color acquired in one predetermined region among the N predetermined regions,

the comparative control frequency distribution is a frequency distribution for the comparative control color acquired in one predetermined region among the N predetermined regions,

in the step of carrying out the shifting, image data corresponding to the N frequency distributions, the first reference frequency distribution, the second reference frequency distribution, and the comparative control frequency distribution are shifted in a direction of one of the at least two color feature quantities with respect to a display region in a case where the N frequency distributions, the first reference frequency distribution, the second reference frequency distribution, and the comparative control frequency distribution are displayed as images, and

each of the first centroid position, the second centroid position, the third centroid position, and the fourth centroid position is a centroid position based on image data corresponding to each of the N frequency distributions, the first reference frequency distribution, the second reference frequency distribution, and the comparative control frequency distribution, which are the shifted image data.

8. The transparency assessment method according to Claim 1, wherein the at least two color feature quantities include hue and saturation.

Fig.1

## Fig.2

*Fig.3*

20

30 { 32
31

10

34

33 { 33b
33a

40

IMAGE PROCESSING DEVICE

INPUT UNIT — 41

CALCULATION UNIT — 42

OUTPUT UNIT — 43

**Fig.4**

# Fig.5

```
                    ┌─────────┐
                    │  START  │
                    └────┬────┘
                         │
    ┌────────────────────────────────────────┐
    │        IMAGE CONVERSION STEP            │ ～S11
    └────────────────────┬───────────────────┘
                         │
    ┌────────────────────────────────────────┐
    │       FREQUENCY DISTRIBUTION            │ ～S12
    │           CREATION STEP                 │
    └────────────────────┬───────────────────┘
                         │
    ┌────────────────────────────────────────┐
    │      FIRST COLOR DETERMINATION STEP     │ ～S13
    └────────────────────┬───────────────────┘
                         │
    ┌────────────────────────────────────────┐
    │           MODEL UPDATE STEP             │ ～S14
    └────────────────────┬───────────────────┘
                         │
    ┌────────────────────────────────────────┐
    │    SECOND COLOR DETERMINATION STEP      │ ～S15
    └────────────────────┬───────────────────┘
                         │
    ┌────────────────────────────────────────┐
    │         AVERAGE FREQUENCY               │ ～S16
    │    DISTRIBUTION CREATION STEP           │
    └────────────────────┬───────────────────┘
                         │
    ┌────────────────────────────────────────┐
    │              SHIFT STEP                 │ ～S17
    └────────────────────┬───────────────────┘
                         │
    ┌────────────────────────────────────────┐
    │           ASSESSMENT STEP               │ ～S18
    └────────────────────┬───────────────────┘
                         │
                    ┌─────────┐
                    │   END   │
                    └─────────┘
```

# Fig.6

13p

P2

Se1p

Se2p

Se3p

Se4p

Se5p

Se6p

Se7p

Se8p

Fig.7

*Fig.8*

*Fig.9*

SATURATION

HUE

52

A2

50

Fig.10

# Fig.11

SATURATION

HUE

50

51

*Fig.12*

Fig.13

# Fig.14

## INTERNATIONAL SEARCH REPORT

| International application No. |
| --- |
| **PCT/JP2023/003087** |

**A. CLASSIFICATION OF SUBJECT MATTER**

*G01N 21/27*(2006.01)i; *C12M 1/34*(2006.01)i; *C12Q 1/06*(2006.01)i; *G01J 3/46*(2006.01)i; *G01N 33/53*(2006.01)i; *G01N 33/574*(2006.01)i

FI: G01N21/27 Z; C12M1/34 B; C12Q1/06; G01N33/574 Z; G01N33/53 Z; G01J3/46 Z

According to International Patent Classification (IPC) or to both national classification and IPC

**B. FIELDS SEARCHED**

Minimum documentation searched (classification system followed by classification symbols)

G01N21/00-G01N1/61; G01N21/75-G01N21/83; C12M1/00-C12M3/10; C12Q1/00-C12Q3/00; G01J3/00-G01J3/52; G01N33/48-G01N33/98

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| A | WO 2021/095381 A1 (SUMITOMO CHEMICAL CO) 20 May 2021 (2021-05-20)<br>entire text, all drawings | 1-8 |
| A | JP 2010-223933 A (FUJI ELECTRIC SYSTEMS CO LTD) 07 October 2010 (2010-10-07)<br>entire text, all drawings | 1-8 |
| A | WO 2013/145873 A1 (SATAKE CORPORATION) 03 October 2013 (2013-10-03)<br>entire text, all drawings | 1-8 |
| A | US 2019/0011252 A1 (ARKEMA INC) 10 January 2019 (2019-01-10)<br>entire text, all drawings | 1-8 |
| A | CN 111368643 A (HANGZHOU DIANZI UNIVERSITY) 03 July 2020 (2020-07-03)<br>entire text, all drawings | 1-8 |

☐ Further documents are listed in the continuation of Box C.　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **28 March 2023** | **11 April 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
### Information on patent family members

International application No.

**PCT/JP2023/003087**

| Patent document cited in search report | | | Publication date (day/month/year) | Patent family member(s) | | | Publication date (day/month/year) |
|---|---|---|---|---|---|---|---|
| WO | 2021/095381 | A1 | 20 May 2021 | US | 2022/0383550 | A1 | |
| | | | | entire text, all drawings | | | |
| | | | | EP | 4056672 | A1 | |
| | | | | KR | 10-2022-0098166 | A | |
| | | | | CN | 114746539 | A | |
| | | | | TW | 202133184 | A | |
| JP | 2010-223933 | A | 07 October 2010 | (Family: none) | | | |
| WO | 2013/145873 | A1 | 03 October 2013 | US | 2015/0076042 | A1 | |
| | | | | entire text, all drawings | | | |
| | | | | EP | 2832458 | A1 | |
| | | | | CN | 104203436 | A | |
| | | | | KR | 10-2014-0145163 | A | |
| | | | | TW | 201400198 | A | |
| US | 2019/0011252 | A1 | 10 January 2019 | US | 2021/0025693 | A1 | |
| | | | | entire text, all drawings | | | |
| | | | | WO | 2017/120160 | A1 | |
| | | | | EP | 3400431 | A1 | |
| | | | | TW | 201733688 | A | |
| | | | | AR | 107318 | A | |
| | | | | CN | 108474739 | A | |
| | | | | MX | 2018008315 | A | |
| CN | 111368643 | A | 03 July 2020 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017067605 A

- JP 2017085966 A

**Non-patent literature cited in the description**

- **FLUCKIGER-ISLER S.** ; **KAMBER M.** Direct comparison of the Ames microplate format (MPF) test in liquid medium with the standard Ames pre-incubation assay on agar plates by use of equivocal to weakly positive test compounds. *Mutation Research/Genetic Toxicology and Environmental Mutagenesis*, 2012, 36-45

- **SUI H.** ; **KAWAKAMI K** ; **SAKURAI N.** ; **HARA T** ; **NOHMI T**. Improvement and evaluation of high throughput fluctuation Ames test using 384-well plate with Salmonella typhimurium TA100 and TA98.. *Genes and Environment*, 2009, vol. 31 (2), 47-55

- **KAMBER M** ; **FLUCKIGER-ISLER S** ; **ENGELHARDT G.** ; **JAECKH R** ; **ZEIGER E**. Comparison of the Ames II and traditional Ames test responses with respect to mutagenicity, strain specificities, need for metabolism and correlation with rodent carcinogenicity. *Mutagenesis*, 2009, vol. 24 (4), 359-366